(19) Europäisches Patentamt
European Patent Office
Office européen des brevets

(11) **EP 1 658 491 B1**

(12) **EUROPÄISCHE PATENTSCHRIFT**

(45) Veröffentlichungstag und Bekanntmachung des Hinweises auf die Patenterteilung:
**31.12.2014 Patentblatt 2015/01**

(21) Anmeldenummer: **04740993.3**

(22) Anmeldetag: **14.07.2004**

(51) Int Cl.:
*G01N 31/16* (2006.01)     *G01N 25/56* (2006.01)
*G01N 33/18* (2006.01)

(86) Internationale Anmeldenummer:
**PCT/EP2004/007777**

(87) Internationale Veröffentlichungsnummer:
**WO 2005/017520 (24.02.2005 Gazette 2005/08)**

(54) **STABILER WASSERSTANDARD**

STABLE WATER STANDARD

ETALON D'EAU STABLE

(84) Benannte Vertragsstaaten:
**AT BE BG CH CY CZ DE DK EE ES FI FR GB GR HU IE IT LI LU MC NL PL PT RO SE SI SK TR**

(30) Priorität: **08.08.2003 DE 10336571**

(43) Veröffentlichungstag der Anmeldung:
**24.05.2006 Patentblatt 2006/21**

(73) Patentinhaber: **Merck Patent GmbH
64293 Darmstadt (DE)**

(72) Erfinder:
• **GRÜNKE, Silke
28201 Bremen (DE)**

• **BAUER, Stefanie
64739 Hoechst i.Odw. (DE)**

(56) Entgegenhaltungen:
**EP-A- 0 482 465     WO-A-02/40991**

• **NEUSS J D ET AL: "SODIUM TARTRATE DIHYDRATE AS A PRIMARY STANDARD FOR KARL FISCHER REAGENT" ANALYTICAL CHEMISTRY, AMERICAN CHEMICAL SOCIETY. COLUMBUS, US, Bd. 23, 1951, Seiten 1332-1333, XP001042005 ISSN: 0003-2700**

**Beschreibung**

Die Erfindung betrifft feste Wasserstandards mit verbesserter Stabilität. Derartige Standards mit definiertem Wassergehalt werden typischerweise als Kalibratoren und/oder Kontrollproben in der Aquametrie unter Verwendung von Ofentechniken eingesetzt. Ein wichtiges Anwendungsfeld ist die Ofentechnik in Kombination mit der Karl-Fischer-Bestimmung (KF-Ofentechnik).

Bei der Karl-Fischer(KF)-Ofentechnik wird die Probe zunächst durch ein frei programmierbares Temperaturprofil aufgeheizt. Ein inertes und trockenes Trägergas, welches durch den Probenraum strömt, nimmt das freigesetzte Wasser auf und transportiert es in die angeschlossene Titrationszelle. In der mit Karl-Fischer-Reagenz befüllten Zelle erfolgt die Wasserbestimmung. Die Bestimmung kann sowohl volumetrisch als auch coulometrisch nach Karl Fischer durchgeführt werden.

Karl-Fischer-Reagenzien enthalten im wesentlichen Jod (Oxidationsmittel), Schwefeldioxid oder dessen Salze (Reduktionsmittel), eine basische Komponente und eine alkoholische Komponente. Bei der Reaktion wird unter Verbrauch des zu bestimmenden Wassers das Reduktionsmittel durch das Oxidationsmittel oxidiert.

Andere Einsatzmöglichkeiten für den festen Wasserstandard sind thermogravimetrische Verfahren oder thermische Verfahren in Kombination mit verschiedenen Sensoren (z.B. coulometrisch auf $P_2O_5$-Basis).

Sowohl zur Qualitätssicherung als auch zur Kalibrierung werden Standardsubstanzen mit definiertem Wassergehalt benötigt. Als Urtitersubstanz mit einem Wassergehalt von 15,66 ($\pm$ 0,10) Gewichtsprozent dient Dinatrium-tartrat-Dihydrat, siehe z.B. WO 02/40991. In EP 0 012 617 wird die Verwendung von Kaolinit ($Al_2[(OH)_4/Si_2O_5]$; $Al_2O_3.2SiO_2.2H_2O$) mit einem Wassergehalt von 15 Gewichtsprozent beschrieben. Bekannt ist auch die Verwendung von Natriumwolframat-dihydrat als Standardsubstanz (Wassergehalt von 11 Gewichtsprozent). Um auch Standards mit niedrigerem Wassergehalt bereitzustellen, werden für diesen Zweck andere Substanzen kommerziell angeboten: Kaliumcitrat-Monohydrat (5,55 Gew.-% Wasser) oder Lactose (5 Gew.-% Wasser). Die Verwendung von Lactose als Standard ist in EP 0 482 465 A1 offenbart. Bevorzugterweise sollte die Kalibrierung eines Messverfahrens in der Nähe des vorgesehenen Arbeitspunktes erfolgen, d.h. mit einem Standard, der einen Wassergehalt aufweist, wie sie in den Messproben zu erwarten ist, z.B. 1 Gew.-% Wasser. Ein derartiger Standard ist kommerziell erhältlich: Er weist einen Wassergehalt von 1 Gew.-% auf und besteht aus einer Mischung von wasserfreiem Natriumwolframat und Natriumwolframat-Dihydrat (siehe Sicherheitsdatenblatt für Art. Nr. 188053; Merck KGaA, Darmstadt, DE). Diese beiden Substanzen lassen in anderen Abmischungen eine Einstellung des Wassergehaltes von 10 Gew.-% Wasser bis etwa 0,1 Gew.-% Wasser zu. Dieser Wasserstandard erfüllt somit wesentliche Anforderungen der Anwender, insbesondere der Methode nach Karl Fischer in Kombination mit einem KF-Ofen:

-   geringer Wassergehalt von 1 %
-   schnelle und vollständige Wasserabgabe bei Temperaturen > 100 °C
-   Anwendung im weiten Temperaturbereich (hohe Temperaturstabilität, keine Zersetzung unter 300 °C).

Mit der Einführung von neuen Geräten für die Ofentechnik (z.B. Oven Sample Processor 774 von Metrohm, Stromboli von Mettler, KF-Ofen von ECH Halle) wird die Nachfrage nach einem solchen Ofen-Standard und die Anforderungen an dessen Qualitätseigenschaften immer größer.

Es stellte sich jedoch heraus, dass bei der Lagerung des oben erwähnten Wasserstandards unter extrem ungünstigen Lager- und Anwendungsbedingungen Probleme bezüglich der Stabilität auftreten. Somit ergibt sich die Aufgabe, einen Wasserstandard mit verbesserter Stabilität bereitzustellen, wobei dessen Wassergehalt je nach verwendeten Komponenten insbesondere im Bereich von 10 Gew.-% Wasser bis etwa 0,1 Gew.-% Wasser eingestellt werden kann, und der seinen Wassergehalt auch bei offener Handhabung oder Lagerung in feuchter Atmosphäre (z.B. größer 60 % relative Feuchte) nur geringfügig ändert.

Es wurde gefunden, dass die Stabilität des oben beschriebenen Wasserstandards, der aus einer Mischung von wasserfreiem Natriumwolframat und Natriumwolframat-Dihydrat besteht, erheblich verbessert werden kann, wenn man anstelle von wasserfreiem Natriumwolframat Kaliumsulfat einsetzt. Im weiteren wurde festgestellt, dass Wasserstandards mit verbesserter Stabilität generell zugänglich sind, in dem man mindestens eine stabile wasserhaltige Verbindung und mindestens eine stabile wasserfreie Verbindung gemäß Anspruch 5 mahlt und mischt.

Gegenstand der Erfindung sind feste, pulverförmige Standards für die Bestimmung von Wasser bestehend aus mindestens einer stabilen wasserhaltigen Verbindung und einer stabilen wasserfreien Verbindung gemäß Anspruch 5, wobei die Bestandteile Partikelgrößen von weniger als 300 $\mu$m aufweisen und homogen Vermischt sind. Bevorzugte Ausführungsformen weisen einen Wassergehalt zwischen 0,005 und 10 Gewichts-% auf. Gegenstand der Erfindung ist weiterhin ein Verfahren zur Herstellung dieser Standards umfassend folgende Arbeitsschritte:

a) Bereitstellen der mindestens einen stabilen wasserhaltigen Verbindung sowie der mindestens einenstabilen wasserfreien Verbindung;

b) Verkleinern der Partikelgröße der in a) genannten Bestandteile auf weniger als 300 $\mu$m;

c) Berechnen der Anteile der stabilen wasserhaltigen Verbindung(en) und der stabilen wasserfreien Verbindung(en), damit in der Mischung der für den Standard gewünschte Wassergehalt resultiert;

d) Mischen der aus Schritt b) erhaltenen Bestandteile entsprechend der in Schritt c) berechneten Anteile,

wobei die Reihenfolge der Schritte b) und c) vertauscht werden kann. Gegenstand der Erfindung ist schließlich die Verwendung einer festen, pulverförmigen Mischung bestehend aus mindestens einer stabilen wasserhaltigen Verbindung und einer stabilen wasserfreien Verbindung als Wasserstandard für die Bestimmung von Wasser mittels Ofentechnik nach Karl-Fischer gemäß Anspruch 1.

Kriterien für die Auswahl der erfindungsgemäß geeigneter Verbindungen sind:

- chemisch reaktionsträge
- ähnliche Schüttdichten
- gute Mischbarkeit
- Schmelzpunkt > 400 °C
- geringes Gefahrenpotential bzgl. Toxizität

Bevorzugt werden dabei anorganische Salze. Die durch die vorliegende Erfindung bereitgestellten Wasserstandards zeichnen sich durch hohe Stabilität auch bei erhöhter Luftfeuchtigkeit aus. Weiterhin können die erfindungsgemäßen Wasserstandards in einen Temperaturbereich zwischen 140 und 400 °C verwendet werden. Die Wasserabgabe erfolgt mit hoher Geschwindigkeit bei Temperaturen über 100 °C. Dadurch wird das Wasser bei Probenmengen unter 0,5 g innerhalb von 5 Minuten aus dem Standard freigesetzt. Es werden Analysenzeiten erreicht, die nicht wesentlich von der Freisetzungskinetik der Wasserabgabe limitiert werden.

Die Schüttdichten der Komponenten sollten nicht zu stark voneinander abweichen. Die zentrale Rolle für den Homogenisierungsgrad spielt jedoch die Korngröße. Sie bestimmt einerseits die Güte der Zufallsmischung und andererseits die Fließeigenschaften, die wiederum die Durchmischung beeinflussen. Allgemein lassen sich Feststoffe ähnlicher Korngröße gut mischen. Bei stark abweichenden Korngrößen treten Schwierigkeiten beim Herstellen einer homogenen Mischung auf. Spätestens bei Transport und Lagerung werden Entmischungseffekte beobachtet. Hauptursache ist hier meist die Bewegung der feinen Teilchen durch die groberen Teilchen (Perkollation). Gesichtspunkte für die Herstellung von Pulvermischungen sind insbesondere dem Fachmann auf dem Gebiet der Galenik bekannt und in einschlägigen Handbüchern, beispielsweise in Hagers Handbuch der pharmazeutischen Praxis, 5. Auflage, Springer Verlag, Berlin-Heidelberg 1991, beschrieben. Auch in Standardnachschlagewerken wie Kirk-Othmer, Encyclopedia of Chemical Technology, 4 th edition, volume 19, John Wiley & sons, NY 1996, S. 1132, sind derartige Angaben zu finden.

Unter der Voraussetzung freifließender Materialien können bereits Korngrößenunterschiede kleiner als 2 : 1 bei mittleren Korngrößen von 100-200 $\mu$m eine Segregation beim Transport von Pulvermischungen verursachen. Bei stark unterschiedlichen Konzentrationsanteilen zweier Substanzen in einer Mischung ist damit nicht nur eine ähnliche sondern auch eine zunehmend geringe Korngröße Voraussetzung für eine homogene Mischung. Für pharmazeutische Pulvermischungen mit geringem Arzneiwirkstoffgehalt werden beispielsweise in Abhängigkeit der einzustellenden Dosis maximale Grenzpartikelgrößen definiert. Weiterhin beeinflussen Kohäsions- und Adhäsionserscheinungen das Verhalten der Mischung. Aber auch die Schüttdichten der zu mischenden Pulver beeinflußen die Qualität der Mischung. Aufgrund der Komplexität der Zusammenhänge können hier aber keine quantitativen Aussagen getroffen werden.

Für die Verwendung bei der Herstellung eines erfindungsgemäßen Wasserstandards werden die Rohstoffe, bevorzugterweise in analysenreiner (p.a.) Qualität gemahlen, so dass die Korngröße weniger als 300 $\mu$m, bevorzugterweise weniger als 150 $\mu$m beträgt. Entsprechend des gewünschten Wassergehaltes des Standards und der Wassergehalte der wasserhaltigen Komponente sowie der wasserfreien Komponente werden die Anteile der wasserhaltigen und der wasserfreien Komponente eingewogen und zur Homogenität vermischt. Vorrichtungen und deren Verwendung zum Mahlen von Salzen oder ähnlichen Feststoffen sind dem Fachmann ebenso bekannt wie Vorrichtungen und Verfahren zum Sichten und Mischen von durch Mahlen erhaltenen Pulvern.

Die Einwaagen ($M_1$, $M_2$) für die beiden Komponenten in Abhängigkeit von deren Wassergehalt ($W_1$, $W_2$) und dem gewünschten Wassergehalt ($W_P$) und der gewünschten Menge des Produktes ($M_P$) lassen sich nach folgenden beiden Gleichungen berechnen:

$$M_1 * W_1 + M_2 * W_2 = M_P * W_P \tag{I}$$

$$M_1 + M_2 = M_P \tag{II},$$

worin der Index 1 die wasserfreie Komponente, der Index 2 die wasserhaltige Komponente und der Index P das Produkt, d.h. den Standard, kennzeichnen. Bei einer gegebenen vorgelegten Einwaage der wasserfreien (bzw. wasserarmen) Komponente berechnet sich die notwendige Menge der wasserhaltigen Komponente folglich nach Formel III:

$$M_2 = M_1 * \frac{(W_P - W_1)}{(W_2 - W_P)} \qquad \text{(III)}$$

Als wasserfreie Komponente sind neben dem bevorzugten Kaliumsulfat grundsätzlich Salze und Metalloxide geeignet, die nur ohne Wasser oder Wassereinschluss kristallisieren, und nicht hygroskopisch sind. Erfindungsgemäß sind dies: Bariumsulfat, Titandioxid (Rutil) und Calciumphosphat. Verwendung können grundsätzlich auch Verbindungen finden, deren wasserfreie Form hinreichend stabil gegenüber wasserhaltigen Formen ist. Anstelle der wasserfreien Komponente sind unter Umständen auch stabile wasserarme Verbindungen geeignet, deren Wassergehalt unter 5 Gewichtsprozent, bevorzugterweise unter 1 Gewichtsprozent beträgt. Wie bereits erwähnt, ist jedoch die Stabilität von wasserfreiem Natriumwolframat in derartigen Standards unzureichend, da die Standards bei ungünstigen Lagerungsverhältnissen (Temperaturen > 25 °C und relative Luftfeuchte > 80 %) Wasser aufnehmen. Die wasserfreie sowie die wasserhaltige Komponente dürfen keine hygroskopischen Eigenschaften haben.

Im Zusammenhang der vorliegenden Erfindung wird eine wasserfreie Komponente als stabil definiert, wenn ihr absoluter Wassergehalt im Verlauf von vier Wochen bei offener Lagerung zwischen 15 und 30 °C und relativen Luftfeuchten zwischen 20 und 80 % um nicht mehr als 0,05 % ansteigt.

Als wasserhaltige Komponente eignen sich grundsätzlich Salze, die als stabile, definierte Hydrate vorliegen. Das Wasser ist in diesen Verbindungen typischerweise in stöchiometrischen Verhältnissen als Kristallwasser gebunden. Als wasserhaltige Komponente geeignete Hydrate werden als thermodynamisch stabil betrachtet, wenn deren Dampfdruck kleiner ist als der Partialdruck des Wasserdampfs in der Luft (allgemein Luftfeuchtigkeit). Ist der Dampfdruck eines Hydrates größer als Partialdruck des Wasserdampfs in der Luft, wird das Kristallwasser an die Luft abgegeben, das Hydrat verwittert. Neben dem bevorzugten Natriumwolframat-Dihydrat eignen sich Salze, bei denen nur eine Hydratform existiert, so dass die Wasserabgabe vollständig bei einer Temperatur erfolgt; erfindungsgemäß ist dies Natriummolybdat-Dihydrat. Im Zusammenhang der vorliegenden Erfindung wird eine wasserhaltige Komponente als stabil definiert, wenn ihr relativer Wassergehalt im Verlauf von vier Wochen bei offener Lagerung zwischen 15 und 30 °C und relativen Luftfeuchten zwischen 20 und 80 % um nicht mehr als 10 % schwankt.

Die Eigenschaften erfindungsgemäßer Standards werden weiter verbessert, wenn die für die Komponenten benutzten Substanzen sich gut auf die angegebene Körnung mahlen lassen. Die gemischten Komponenten sollten ferner frei fließende Pulver bilden und dürfen nicht klumpen. Bei sehr kleinen Korngrößen werden keine frei fließenden Pulver erhalten, jedoch sorgen Adhäsionskräfte für den Ausschluss von Entmischungserscheinungen.

Die bevorzugte Mischung aus Kaliumsulfat und Natriumwolframat-Dihydrat zeigte die besten Eigenschaften. Sie nimmt auch bei offener Lagerung und erhöhter Luftfeuchtigkeit keine Feuchtigkeit auf.

Weiterhin ist bekannt, dass die Entmischung von Pulvermischungen durch zusätzliche Arbeitsschritte wie beispielsweise der Granulation vermieden werden kann.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen; der beanspruchte Schutzumfang wird durch die Ansprüche definiert.

**Beispiele**

**Beispiel 1: Herstellung eines erfindungsgemäßen Wasserstandards**

a) Kaliumsulfat zur Analyse (Art. Nr. 105153; Merck KGaA, Darmstadt, DE), sowie Natriumwolframat-Dihydrat zur Analyse (Art. Nr. 106673; Merck KGaA, Darmstadt, DE) wurden jeweils in einer Feinprallmühle (Fa. alpine Typ 100 UPZ) gemahlen, bis die Partikelgröße 50 $\mu$m oder weniger betrug.

b) Der Wassergehalt der beiden Salze wurde mit Hilfe der Karl-Fischer-Ofentechnik bestimmt; Kaliumsulfat: Einwaage: 0,5 g; Wassergehalt: 0,03 Gew.-% Natriumwolframat-Dihydrat: Einwaage: 0,05 g; Wassergehalt: 10,8 Gew.-%.

c) In einem Pulvermischer (Fa. WAB, Typ Turbula) wurden 4550 g Kaliumsulfat aus Schritt a) vorgelegt und 450 g Natriumwolframat-Dihydrat zur Analyse aus Schritt a) eingetragen und gemischt, bis die Mischung homogen war.

Es resultierte eine als Wasserstandard geeignete Pulvermischung mit einem Wassergehalt von 1,0 Gew.-%.

**Beispiel 2: Herstellung eines Wasserstandards entsprechend dem Stand der Technik (Vergleichsversuch)**

a) Natriumwolframat-Dihydrat zur Analyse (Art. Nr. 106673; Merck KGaA, Darmstadt, DE) wurde durch Trocknen über mehrere Stunden bei 180 °C vom Kristallwasser befreit. Das resultierende wasserfreie Salz wurde in einer Schlagkreuzmühle (Fa. Alpine Typ 100 P) gemahlen, bis die Partikelgröße 150 $\mu$m oder weniger betrug. Ebenso wurde unbehandeltes Natriumwolframat-Dihydrat zur Analyse (Art. Nr. 106673; Merck KGaA, Darmstadt, DE) in einer Schlagkreuzmühle (Fa. Alpine Typ 100 P) gemahlen, bis die Partikelgröße 150 $\mu$m oder weniger betrug.

Ebenso wie in Beispiel 1 beschrieben wurde der Wassergehalt der beiden Salze bestimmt und entsprechende Mengen in einem Pulvermischer gemischt. Es resultierte eine als Wasserstandard geeignete Pulvermischung mit einem Wassergehalt von 1,0 Gew.-%.

**Beispiel 3: Vergleichsversuch Lagerstabilität**

Aliquots von ca. 100 g der nach den Beispielen 1 und 2 erhaltenen Materialien wurden jeweils in 1 Pulverflasche aus Polyethylen mit Schraubverschluss (F) sowie Aliquots von 1 g in jeweils 3 offenen Probegläschen (O) gelagert. Der Wassergehalt wurde jeweils nach der Herstellung und nach 24 sowie nach 28 Wochen mittels der Karl-Fischer-Ofentechnik bestimmt. Pro Standardsubstanz wurden jeweils 3 Bestimmungen (jeweils 1 Probe pro offenem Gefäß (O) und eine Dreifachbestimmung aus der geschlossenen Flasche (F)) durchgeführt.
Gerät: 774 KF oven processor und 756 KF Coulometer mit Zelle mit Diaphragma
Parameter: für Ofen: T = 150 °C, Flussrate 70 mL / min
für Coulometer: Extraktionszeit 300 sec, relative stop Drift <
20 $\mu$g/min, automatische Driftkorrektur
Einwaage: 50 - 200 mg
Die Lagerung erfolgte bei Raumtemperatur. Während der ersten Zeitperiode betrug die Raumtemperatur zwischen 18 und 25 °C bei einer relativen Luftfeuchtigkeit von unter 60 %. Anschließend betrug die Raumtemperatur bis zu 30 °C bei einer Luftfeuchtigkeit bis zu 80 %. Die Ergebnisse sind in der folgenden Tabelle 1 zusammengefasst.

Tabelle 1: Vergleichsversuch Lagerstabilität

| Material / Aufbewahrung | Anfangswert | 24 Wochen | 28 Wochen |
|---|---|---|---|
| Beispiel 1; | 1,0386 | 1,0885 | 1,0471 |
| | 1,0342 | 1,0977 | 1,0547 |
| Lagerung F | 1,0266 | 1,0988 | 1,0535 |
| **Mittelwert:** | **1,0331** | **1,0950** | **1,0518** |
| **(%)** | **100,0** | **106,0** | **101,8** |
| Beispiel 1; | 1,0386 | 1,0668 | 1,0741 |
| | 1,0342 | 1,0368 | 1,0581 |
| Lagerung O | 1,0266 | 1,0353 | 1,0594 |
| **Mittelwert:** | **1,0331** | **1,0463** | **1,0639** |
| **(%)** | **100,0** | **101,3** | **103,0** |
| Beispiel 2 (SdT); | 0,9893 | 0,9965 | 0,9604 |
| | 0,9999 | 0,9683 | 0,9679 |
| Lagerung F | 0,9905 | 0,9842 | 0,9751 |
| **Mittelwert:** | **0,9932** | **0,9830** | **0,9665** |
| **(%)** | **100,0** | **99,0** | **97,3** |
| Beispiel 2 (SdT); | 0,9893 | 1,0052 | 2,1661 |
| | 0,9999 | 1,0217 | 2,1790 |
| Lagerung O | 0,9905 | 1,0463 | 2,1120 |

(fortgesetzt)

| Material / Aufbewahrung | Anfangswert | 24 Wochen | 28 Wochen |
|---|---|---|---|
| Mittelwert: | 0,9932 | 1,0244 | 2,1524 |
| (%) | 100,0 | 103,1 | 216,7 |

**Beispiel 4: Homogenität der Mischung**

Die nach Beispiel 1 hergestellte Mischung wurde in einer gefüllten 100 mL PE-Flasche 20 min auf einer Schüttelmaschine gerüttelt und danach auf eventuell auftretende Entmischungseffekte untersucht. Dazu wurden aus der oberen und unteren Fraktion jeweils 5 Proben zur Wasserbestimmung entnommen. Es konnten keine Entmischungserscheinungen festgestellt werden.

Parameter für die Bestimmung: siehe Beispiel 3

Ergebnisse:

Obere Fraktion: (0,967 +/- 0,002) % Wasser
Untere Fraktion: (0,968 +/- 0,006) % Wasser

**Patentansprüche**

1. Verwendung einer Mischung für die Bestimmung von Wasser mittels Ofentechnik nach Karl-Fischer, enthaltend mindestens

    a) eine thermodynamisch stabile wasserhaltige Verbindung, ausgewählt aus der Gruppe Natriumwolframat-Dihydrat und Natriummolybdat-Dihydrat, Natriummolybdat-Dihydrat, und
    b) eine stabile wasserfreie Verbindung, ausgewählt aus der Gruppe Kaliumsulfat, Bariumsulfat, Titandioxid (Rutil) und Calciumphosphat
    als Wasserstandard für die Bestimmung von Wasser mit einem Wassergehalt zwischen 0,005 und 10 Gewichts-%,
    wobei die Komponenten a) und b) Korngrößen von weniger als 300 $\mu$m aufweisen und homogen vermischt sind.

2. Verwendung einer Mischung nach Anspruch 1, worin die Komponenten a) und b) Korngrößen von weniger als 50 $\mu$m aufweisen und homogen vermischt sind.

3. Verwendung einer Mischung nach Anspruch 1 oder 2, deren Komponenten a) und b) nach dem homogenen Vermischen einer Granulation unterzogen werden.

4. Verwendung einer Mischung nach einem oder mehreren der Ansprüche 1 bis 3, enthaltend eine homogene Mischung aus Natriumwolframat-Dihydrat und Kaliumsulfat.

5. Standard für die Bestimmung von Wasser mittels Ofentechnik nach Karl-Fischer, enthaltend mindestens

    a) eine thermodynamisch stabile wasserhaltige Verbindung, ausgewählt aus der Gruppe Natriumwolframat-Dihydrat und/ Natriummolybdat-Dihydrat,
    und
    b) eine stabile wasserfreie Verbindung, ausgewählt aus der Gruppe Kaliumsulfat, Bariumsulfat, Titandioxid (Rutil) und Calciumphosphat,

wobei die Komponenten a) und b) Korngrößen von weniger als 300 $\mu$m aufweisen und homogen vermischt sind.

6. Standard nach Anspruch 5 mit einem Wassergehalt zwischen 0,005 und 10 Gewichts-%.

7. Verfahren zur Herstellung eines Wasserstandards nach einem der Ansprüche 5 oder 6, umfassend die folgenden Schritte:

    a) Bereitstellen der mindestens einen stabilen wasserhaltigen Verbindung sowie der mindestens einen stabilen wasserfreien Verbindung;

b) Verkleinern der Partikelgröße der in a) genannten Bestandteile auf weniger als 300 μm;

c) Berechnen der Anteile der stabilen wasserhaltigen Verbindung(en) und der stabilen wasserfreien Verbindung(en), damit in der Mischung der für den Standard gewünschte Wassergehalt resultiert;

d) Mischen der aus Schritt b) erhaltenen Bestandteile entsprechend der in Schritt c) berechneten Anteile, wobei die Reihenfolge der Schritte b) und c) vertauscht werden kann.

**Claims**

**1.** Use of a mixture for the determination of water by means of the Karl Fischer oven technique, comprising

a) at least one thermodynamically stable water-containing compound selected from the group sodium tungstate dihydrate and sodium molybdate dihydrate,

and

b) at least one stable water-free compound selected from the group potassium sulfate, barium sulfate, titanium dioxide (rutile) and calcium phosphate,

as water standard for the determination of water having a water content between 0.005 and 10% by weight, where components a) and b) have particle sizes of less than 300 μm and are homogeneously mixed.

**2.** Use of a mixture according to Claim 1 in which components a) and b) have particle sizes of less than 150 μm and are homogeneously mixed.

**3.** Use of a mixture according to Claim 1 or 2 whose components a) and b) are subjected to granulation after the homogeneous mixing.

**4.** Use of a mixture according to one or more of Claims 1 to 3, comprising a homogeneous mixture of sodium tungstate dihydrate and potassium sulfate.

**5.** Standard for the determination of water by means of the Karl Fischer oven technique, comprising

a) at least one thermodynamically stable water-containing compound selected from the group sodium tungstate dihydrate and sodium molybdate dihydrate,

and

b) at least one stable water-free compound selected from the group potassium sulfate, barium sulfate, titanium dioxide (rutile) and calcium phosphate,

where components a) and b) have particle sizes of less than 300 μm and are homogeneously mixed.

**6.** Standard according to Claim 5 having a water content between 0.005 and 10% by weight.

**7.** Process for the preparation of a water standard according to one of Claims 5 or 6, comprising the following steps:

a) provision of the at least one stable water-containing compound and the at least one stable water-free compound;

b) reduction of the particle size of the constituents mentioned in a) to less than 300 μm;

c) calculation of the proportions of the stable water-containing compound(s) and of the stable water-free compound(s) in order that the water content desired for the standard results in the mixture;

d) mixing of the constituents obtained from step b) in accordance with the proportions calculated in step c),

where the sequence of steps b) and c) can be reversed.

**Revendications**

**1.** Utilisation d'un mélange pour la détermination de l'eau à l'aide de la technique du four de Karl Fischer, comprenant

a) au moins un composé contenant de l'eau et thermodynamiquement stable, choisi dans le groupe constitué par le tungstate de sodium dihydraté et le molybdate de sodium dihydraté,
et
b) au moins un composé dépourvu d'eau et stable choisi dans le groupe constitué par le sulfate de potassium, le sulfate de baryum, le dioxyde de titane (rutile) et le phosphate de calcium,

comme étalon d'eau pour la détermination de l'eau ayant une teneur en eau comprise entre 0,005 et 10% en poids, où les composants a) et b) possèdent des tailles de particules inférieures à 300 $\mu$m et sont mélangés de manière homogène.

2. Utilisation d'un mélange selon la revendication 1, dont les composants

a) et b) possèdent des tailles de particules inférieures à 150 $\mu$m et sont mélangés de manière homogène.

3. Utilisation d'un mélange selon la revendication 1 ou 2, dont les composants a) et b) sont soumis à une granulation après le mélange homogène.

4. Utilisation d'un mélange selon l'une ou plusieurs parmi les revendications 1 à 3, comprenant un mélange homogène de tungstate de sodium dihydraté et de sulfate de potassium.

5. Etalon pour la détermination de l'eau à l'aide de la technique du four de Karl Fischer, comprenant

a) au moins un composé contenant de l'eau et thermodynamiquement stable, choisi dans le groupe constitué par le tungstate de sodium dihydraté et le molybdate de sodium dihydraté,
et
b) au moins un composé dépourvu d'eau et stable choisi dans le groupe constitué par le sulfate de potassium, le sulfate de baryum, le dioxyde de titane (rutile) et le phosphate de calcium,

où les composants a) et b) possèdent des tailles de particules inférieures à 300 $\mu$m et sont mélangés de manière homogène.

6. Etalon selon la revendication 5, possédant une teneur en eau comprise entre 0,005 et 10% en poids.

7. Procédé de préparation d'un étalon d'eau selon l'une des revendications 5 ou 6, comprenant les étapes suivantes :

a) la fourniture du au moins un composé contenant de l'eau et stable et du au moins un composé dépourvu d'eau et stable;
b) la réduction de la taille de particules des constituants mentionnés dans a) jusqu'à moins de 300 $\mu$m;
c) le calcul des proportions du ou des composés contenant de l'eau et stables et du ou des composés dépourvus d'eau et stables afin que la teneur en eau désirée pour l'étalon apparaisse dans le mélange ;
d) le mélange des constituants obtenus dans l'étape b) conformément aux proportions calculées dans l'étape c),

où la séquence des étapes b) et c) peut être inversée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0240991 A
- EP 0012617 A
- EP 0482465 A1

**In der Beschreibung aufgeführte Nicht-Patentliteratur**

- **HAGERS.** Handbuch der pharmazeutischen Praxis. Springer Verlag, 1991
- **KIRK-OTHMER.** Encyclopedia of Chemical Technology. John Wiley & sons, 1996, vol. 19, 1132